# FASCICULE DE BREVET EUROPEEN

(11) **EP 2 252 286 B1**
(45) Date de publication et mention de la délivrance du brevet: **24.08.2011**
(21) Numéro de dépôt: 09721879.6
(22) Date de dépôt: 05.02.2009
(51) Int. Cl.: A61K 31/357

(54) **ASSOCIATION ENTRE UN SEL DE BIS-THIAZOLIUM OU L'UN DE SES PRECURSEURS ET L'ARTEMISININE OU L'UN DE SES DERIVES POUR LE TRAITEMENT DU PALUDISME SEVERE**
KOMBINATION AUS EINEM BIS-THIAZOL-SALZ ODER EINEM VORLÄUFER DAVON UND ARTEMISININ ODER EINEM DERIVAT DAVON ZUR BEHANDLUNG AKUTER MALARIA
COMBINATION OF A BIS-THIAZOLIUM SALT OR A PRECURSOR THEREOF AND ARTEMISININ OR A DERIVATIVE THEREOF FOR TREATING ACUTE MALARIA

(30) Priorité: 06.02.2008 FR 0800618
(43) Date de publication de la demande: 24.11.2010
(73) Titulaire: SANOFI, 75013 Paris (FR); Centre National de la Recherche Scientifique (CNRS), 75794 Paris Cedex 16 (FR); University of Montpellier II, 34095 Montpellier Cedex (FR)
(72) Inventeur: FRAISSE, Laurent, F-75013 Paris (FR); VIAL, Henri, F-75794 Paris Cedex 16 (FR); WEIN, Sharon Aurore, F-75794 Paris cedex 16 (FR)
(74) Mandataire: Werner, Alain Henri
(86) Numéro de dépôt international: PCT/FR2009/000129
(87) Numéro de publication internationale: WO 2009/115666

(56) Documents cités:
- FR-A- 2 796 642
- FR-A- 2 884 715
- GELB ET AL: "Drug discovery for malaria: a very challenging and timely endeavor" CURRENT OPINION IN CHEMICAL BIOLOGY, CURRENT BIOLOGY LTD, LONDON, GB, vol. 11, no. 4, 27 août 2007 (2007-08-27), pages 440-445, XP022216091 ISSN: 1367-5931
- TAUDON ET AL: "Quantitative analysis of a bis-thiazolium antimalarial compound, SAR97276, in mouse plasma and red blood cell samples, using liquid chromatography mass spectrometry" JOURNAL OF PHARMACEUTICAL AND BIOMEDICAL ANALYSIS, NEW YORK, NY, US, vol. 46, no. 1, 13 octobre 2007 (2007-10-13), pages 148-156, XP022382363 ISSN: 0731-7085
- RICHIER ERIC ET AL: "Potent antihematozoan activity of novel bisthiazolium drug T16: evidence for inhibition of phosphatidylcholine metabolism in erythrocytes infected with Babesia and Plasmodium spp." ANTIMICROBIAL AGENTS AND CHEMOTHERAPY OCT 2006, vol. 50, no. 10, octobre 2006 (2006-10), pages 3381-3388, XP002493564 ISSN: 0066-4804
- HAMZÉ ABDALLAH ET AL: "Mono- and bis-thiazolium salts have potent antimalarial activity." JOURNAL OF MEDICINAL CHEMISTRY 19 MAY 2005, vol. 48, no. 10, 19 mai 2005 (2005-05-19), pages 3639-3643, XP002493565 ISSN: 0022-2623

## Description

La présente invention se rapporte à une nouvelle association de principes actifs antipaludiques, à savoir un sel de bis-thiazolium ou l'un de ses précurseurs et l'artémisinine ou ses dérivés, ainsi qu'à une composition pharmaceutique comprenant une telle association, utile pour le traitement et/ou la prévention du paludisme sévère.

Le paludisme est l'une des premières causes infectieuses de mortalité au monde et touche annuellement plus de 500 millions de personnes, parmi lesquelles 1,5 à 3 millions décèdent chaque année. Ce fléau touche principalement l'Afrique sub-saharienne, l'Asie du Sud-Est et l'Amérique Latine.
Quatre types de parasites du genre *Plasmodium* (*P. falciparum, P. malariae, P. vivax* et *P. ovale*), véhiculés par les moustiques Anophèles, propagent le paludisme. *Plasmodium falciparum,* largement répandu en Afrique, est le parasite le plus virulent et est responsable de la plupart des formes mortelles de la maladie.
La forte recrudescence de la maladie observée depuis quelques années est due à plusieurs facteurs, parmi lesquels la résistance de nombreuses souches de *Plasmodium falciparum* aux médicaments actuellement utilisés, tels que la chloroquine, la méfloquine, l'amodiaquine ou encore les antifoliques et antifoliniques tel que la pyriméthamine et la sulfadoxine.

Beaucoup d'individus atteints de paludisme présentent une infection aiguë, avec peu de signes physiques révélateurs à part une anémie légère et une augmentation de volume de la rate (splénomégalie). Les symptômes dépendent de la variété de paludisme. Néanmoins, les caractères cliniques généraux sont : malaise, hyperthermie (fièvre), céphalées, troubles digestifs tels que nausées, vomissements et/ou douleurs abdominales, diarrhée, asthénie, douleurs des muscles, arthralgies (douleurs des articulations) et ictère, notamment.
Le traitement s'effectue en général par l'administration de chloroquine (ou de chlorhydrate basique de quinine en cas de résistance à la chloroquine) jusqu'à obtention d'une amélioration et disparition des parasites dans le sang (entre 3 et 5 jours habituellement). Le patient prend généralement ensuite un médicament unique constitué de 1,5 grammes de sulfadoxine et de 75 mg de pyrirnéthamine.
D'autres traitements à base d'anti-foliques et d'anti-foliniques, ou encore à base de malarone, sont également utilisés.

Le paludisme sévère à *Plasmodium falciparum* est un paludisme cérébral associant une élévation importante de la température (40°C) et un coma de mauvais pronostic malgré le traitement et pour lequel la mortalité s'élève parfois à 20 % chez les adultes et 15 % chez les enfants. L'apparition d'un paludisme sévère est soit progressive soit brutale. Il débute après des convulsions instantanées et passagères d'un ou plusieurs muscles, suivies de décontractions. Elles sont localisées ou généralisées à l'ensemble du corps. Cette variété de paludisme s'accompagne d'un nystagmus (tressautement des yeux dans le plan horizontal de façon incessante), quelquefois d'une raideur du cou et d'une perturbation des réflexes. Le paludisme sévère peut s'accompagner également d'hémorragies de la rétine, d'hypoglycémie, d'oedèmes pulmonaires, d'atteinte des reins, d'anémie, et/ou d'hématémèse (rejet de sang provenant des organes lésés et qui est dégluti, puis réapparaît sous forme d'une hémorragie accompagnant le vomissement).
Dans le cas de *Plasmodium falciparum* causant un paludisme compliqué ou paludisme cérébral ainsi que les autres manifestations sévères, il s'agit d'urgence médicale quand 1 % des hématies (globules rouges) ou plus sont parasités chez un sujet qui n'est pas encore immunisé contre cette maladie.
Le traitement dans le cas du paludisme sévère a pour priorité de faire baisser de manière massive et rapide (dans les 24 à 48 heures suivant la prise en charge) la parasitémie pour écarter le pronostic létal. Une fois la parasitémie maitrisée et le pronostic vital confirmé, un traitement anti-paludique plus classique, tel que chloroquine ou chlorhydrate basique de quinine par exemple, peut être administré.
Le traitement du paludisme sévère est difficilement administrable par voie orale ou rectale, les patients étant souvent atteints de vomissements accompagnés de diarrhées importantes.

Isolée en 1972 de la plante *Artemisia annua* (qinghaosu), utilisée depuis des siècles en Chine, l'artémisinine présente une puissante activité antipaludique. Des dérivés aux propriétés pharmacologiques améliorées, tels que l'artéméther, l'artééther et l'artésunate sont aussi commercialisés.
L'artémisinine et ses dérivés, en particulier l'artésunate, font aujourd'hui partie des principes actifs les plus efficaces contre *Plasmodium falciparum.* Cependant, ces composés conduisent difficilement à des guérisons complètes et on note de nombreuses recrudescences. L'utilisation d'artésimine ou de ses dérivés en monothérapie pourrait donc être un facteur causal de sélection de souches parasitaires résistantes.
La communauté scientifique préconise maintenant l'utilisation d'associations de principes actifs, et en particulier d'associations de l'artémisine ou de ses dérivés avec d'autres principes actifs antipaludiques. Ces polythérapies, nommées ACT (Artemisinin-based Combination Therapies), sont recommandées depuis 2002 par l'Organisation Mondiale de la Santé (OMS). Elles offrent de multiples avantages : amélioration de l'efficacité thérapeutique sur les souches résistantes, protection mutuelle des deux principes actifs contre l'apparition de résistance, réduction de la transmission de la maladie et de la propagation des résistances.
Plusieurs associations de ce type sont connues.
L'association entre l'artéméther et la luméfantrine, commercialisée sous la dénomination Coartem^{®}, a par exemple été proposée, ainsi que l'association entre l'artésunate et l'amodiaquine (Arsucam^{®}).
L'association de la ferroquine avec un dérivé d'artémisinine est décrite dans le document WO 2006/111647.
Cependant, l'effet bénéfique des polythérapies ACT n'est ni évident ni prévisible. Par exemple, il est exposé dans la publication de C. Snyder et al., Experimental Parasitology 115 (2007), 296-300, que l'association de la pipéraquine avec le composé OZ277 semble plus prometteuse que l'association de la pipéraquine avec un dérivé d'artémisinine tel que l'arthéméther.
De même, l'association entre la chloroquine et l'artésunate n'atteint pas des niveaux d'efficacité satisfaisants (Am. J. Trop. Med. Hyg., 2003, 69(1), 19-25 et Transactions of the Royal Society of Tropical Medicine and Hygiene, 2003, 97, 429-433) et peut conduire à la sélection de souches résistantes, notamment à la chloroquine.
Ainsi, même si la stratégie ACT est recommandée par l'OMS, la recherche de nouvelles associations de principes actifs antipaludiques n'en est pas pour autant évidente et doit être poursuivie.

La présente invention concerne une association entre un sel de bis-thiazolium, composé de formule (VI) ou l'un de ses précurseurs tels que définis ci-dessous, et l'artémisine ou l'un de ses dérivés. Parmi les dérivés de l'artémisinine utiles dans la présente invention on peut par exemple citer l'artésunate, l'artéméther, l'artééther, l'hydroartémisinine ou la dihydroartémisinine.
Une telle association montre une synergie intéressante et non prévisible et, par conséquent, est utile pour la prévention et/ou le traitement du paludisme sévère.
Les composés de formule (VI), ainsi que ses précurseurs au sens de la présente invention, sont décrits dans le brevet publié sous le numéro EP 1 196 371. Les précurseurs des sels de bis-thiazolium de formule (VI) au sens de la présente invention répondent aux formules (I), (II), (III), (IV) et (V) de EP 1 196 371. Ce brevet décrit en effet des précurseurs de drogues à effet anti-paludique, caractérisés en ce qu'il s'agit de produits capables de générer des sels de bis-ammonium quaternaire et qu'ils répondent à la formule générale (I) : 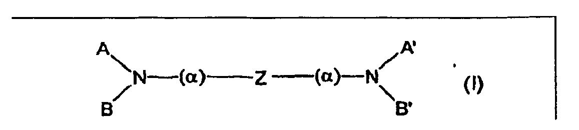 dans laquelle
- A et A' sont identiques ou différents l'un de l'autre et représentent :
   - soit, respectivement, un groupe A₁ et A'₁ de formule : 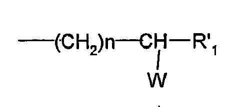 où n est un entier de 2 à 4 ; R'₁ représente un atome d'hydrogène, un radical alkyle en C1 à C5, éventuellement substitué par un radical aryle (notamment un radical phényle), un hydroxy, un alkoxy, dans lequel le radical alkyle comprend de 1 à 5 atomes de carbone, ou aryloxy (notamment phénoxy) ;
      et W représente un atome d'halogène choisi parmi le chlore, le brome ou l'iode, ou un groupe nucléofuge, comme le radical tosyle CH₃-C₆H₄-SO₃, mésityle CH₃-SO₃, CF₃-SO₃, NO₂-CeH₄-SO₃,
   - soit un groupe A₂ qui représente un radical formyle -CHO, ou acétyle -CO-CH₃,
- B et B' sont identiques ou différents l'un de l'autre et représentent :
   - soit respectivement les groupes B₁ et B'₁, si A et A' représentent respectivement A₁ et A'₁, B₁ et B'₁ représentant un groupe R₁ qui présente la même définition que R'₁ ci-dessus, mais ne peut pas être un atome d'hydrogène,
   - soit respectivement les groupes B₂ et B'₂, si A et A' représentent A₂, B₂ ou B'₂ étant le groupe R₁ tel que défini ci-dessus, ou un groupement de formule : 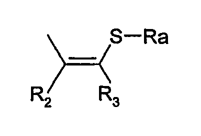
   dans lequel -Ra représente un groupe RS- ou RCO-, où R est un radical alkyle en C1 à C6, notamment de C1 à C5, linéaire, ramifié, ou cyclique, le cas échéant substitué par un ou plusieurs groupes hydroxy, alcoxy ou aryloxy, ou un groupe amino et/ou un groupe - COOH ou COOM, où M est un alkyle en C1 à C3 ; un radical phényle ou benzyle, dans lequel le radical phényle est le cas échéant substitué par au moins un radical alkyle ou alcoxy en C1 à C5, ceux-ci étant éventuellement substitués par un groupe amino, ou par un hétérocycle azoté ou oxygéné, un groupe -COOH ou -COOM ; ou un groupe -CH₂-hétérocycle, à 5 ou 6 éléments, azoté et/ou oxygéné ;
   R₂ représente un atome d'hydrogène, un radical alkyle en C1 à C5, ou un groupe -CH₂-COO-alkyl(C1 à C5);
   et R₃ représente un atome d'hydrogène, un radical alkyle ou alkényle en C1 à C5, le cas échéant substitué par -OH, un groupement phosphate, un radical alkoxy, dans lequel le radical alkyle est en C1 à C3, ou aryloxy; ou un groupe alkyl (ou aryl)carbonyloxy ;
   ou encore R₂ et R₃ forment ensemble un cycle à 5 ou 6 atomes de carbone ; R et R₃ peuvent être reliés pour former un cycle à 5 à 7 atomes (carbone, oxygène, soufre)
- α représente :
   - soit une simple liaison, lorsque A et A' représentent A1 et A'1 : ou lorsque A et A' représentent A₂, c'est-à-dire un groupe -CHO ou -COCH₃, et B₂ et B'₂ représentent : 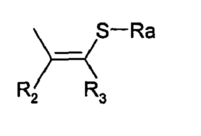
   - soit, lorsque A et A' représentent A₂ et B₂ et B'₂ représentent R₁, un groupement de formule : 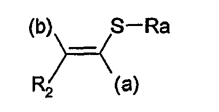 ou un groupement de formule : 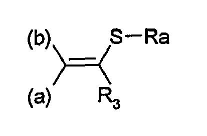
   dans lesquels (a) représente une liaison vers Z et (b) une liaison vers l'atome d'azote,
- Z représente un radical alkyle en C6 à C21, notamment de C12 ou C13 à C21, le cas échéant avec insertion d'une ou de plusieurs liaisons multiples, et/ou d'un ou plusieurs hétéroatomes O et/ou S, et/ou d'un ou de plusieurs cycles aromatiques, et les sels pharmaceutiquement acceptables de ces composés, sous réserve que R'₁ ne représente pas H ou un radical alkyle en C1 ou C2, lorsque n = 3 ou 4, R₁ représente un radical alkyle en C1 à C4 et Z représente un radical alkyle en C6 à C10.
EP 1 196 371 décrit également un sous groupe de composés précurseurs tels que précédemment décrits, répondant à la formule générale (II) : 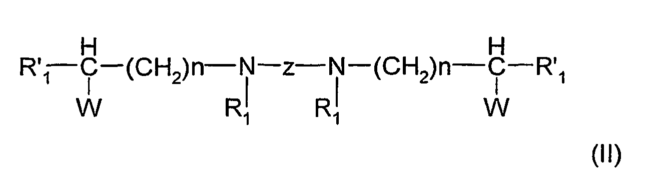 dans laquelle R₁, R'₁, W, n et Z sont tels que définis précédemment.

EP 1 196 371 décrit aussi un autre sous groupe de composés précurseurs, constitué par les précurseurs tels que précédemment décrits, pour lesquels Z représente un radical alkyle de C13 à C21.

EP 1 196 371 décrit également un sous groupe de composés précurseurs, constitué par les précurseurs tels que décrits dans le sous-groupe précédent, pour lesquels Z représente un groupe -(CH₂)₁₆ -.

EP 1 196 371 décrit en particulier les précurseurs choisis parmi le dichlorhydrate du N, N'-diméthyl-N,N'-(5-chloropentyl)-1,16-hexadécanediamine, ou le dichlorhydrate du N, N'-diméthyl-N,N'-(4-chloropentyl)-1,16-hexadécanediamine.

EP 1 196 371 décrit en outre un groupe de précurseurs, répondant à la formule générale (III) : 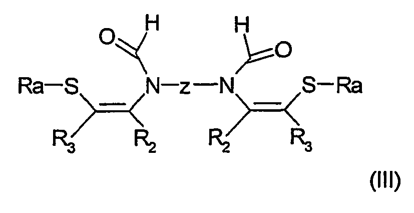 ou à la formule générale (IV) : 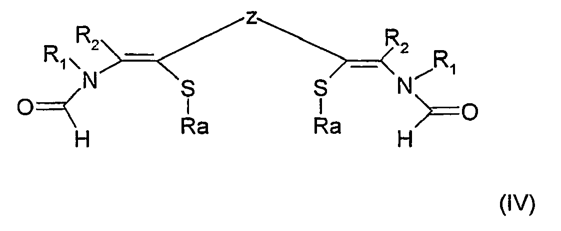 ou à la formule générale (V) : 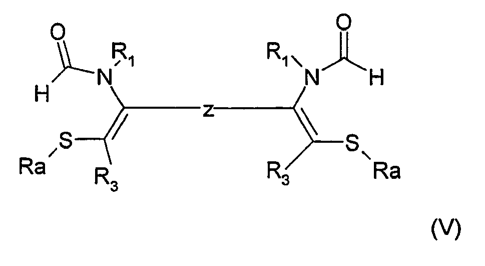 dans laquelle Ra, R₁, R₂, R₃ et Z sont tels que définis précédemment.

EP 1 196 371 décrit en particulier des composés précurseurs de formule (III), (IV) et (V) tels que décrit précédemment choisis parmi :
- N,N'-diformyl-N,N'-di[1-méthyl-2-S-thiobenzoyl-4-méthoxybut-1-ényl]-1, 12-diaminododécane (TE4c),
- N,N'-diformyl-N,N'-di [1-méthyl-2-S-(p-diéthylaminométhylphényl-carboxy)thio-4-méthoxybut-1-ényl]-1, 12-diaminododécane (TE4f),
- N,N'-diformyl-N,N'-di[1-méthyl-2-S-(p-morpholino-méthylphényl carboxy)-thio-4-méthoxybut-1-ényl]-1,12-diaminododécane (TE4g),
- N,N'-diformyl-N,N'-di[1-méthyl-2-S-thiobenzoyl-4-méthoxybut-1-ényl]-1,16- diamino hexadécane (TE8),
- N,N'-diformyl-N,N'-di[1(2-oxo-4,5-dihydro-1,3-oxathian-4-ylidène)éthyl]1,12-diaminododécane (TE3) ;
ou encore parmi :
- N,N'-diformyl-N,N'-di[1-méthyl-2-tétrahydrofurfuryl-méthyldithio-4-hydroxybut-1-ényl]-1,12-diaminododécane (TS3a),
- N,N'-diformyl-N,N'-di[1-méthyl-2-propyl-dithio-4-hydroxybut-1-ényl]-1,12-diaminododécane (TS3b),
- N,N'-diformyl-N,N'-di[1-méthyl-2-benzyl-dithio-4-hydroxybut-1-ényl]-1,12 diaminododécane (TS3c),
- N,N'-diformyl-N,N'-di[1-méthyl-2-(2-hydroxyéthyl)dithio-4-hydroxybut-1-ényl]-1,12-diaminododécane (TS3d),
- N,N'-diformyl-N,N-di[1-méthyl-2-propyldithio-4-méthoxybut-1-ényl]-1,12-diaminododécane (TS3d), et
- N,N'-diformyl-N,N'-di[1-méthyl-2-propyldithio-éthényl]-1,12-diaminododécane (TS6b),
ou bien ;
- 2,17-(N,N'-diformyl-N,N'-diméthyl)diamino-3,16-S-thio-p-méthoxybenzoyl-6,13-dioxaoctadéca-2,16-diéne (TES),
- 2,17-(N,N'-diformyl-N,N'-dibenzyl)amino-3,16-S-thio-p-méthoxybenzoyl-6,13-dioxaoctadéca-2,16-diène (TE10),
- 3,16 (N,N'-diformyl-N,N'-diméthyldiamino-4,17-S-thiobenzoyl-eicosa-3,17-diénedioate d'éthyle (TE12),
- 3,18-(N,N'-diformyl-N,N'-dibenzyl)diamino-4,17-S-thiobenzoyl-eicosa-3,17-diènedioate d'éthyle (TE13),
   ou enfin:
- le 2,15-(N,N'-diformyl-N,N'-diméthyl)diamino-1,16-S-thiobenzoyl-hexadéca-1,16-diéne (TE15),
- le 2,15-(N,N'-diformyl-N,N'-dibenzyl)diamino-1,16-S-thio-benzoyl-hexadéca-1,15-diéne (TE16),

EP 1 196 371 décrit de plus des dérivés cyclisés générés à partir des précurseurs de sels de thiazolium, présentant une bonne activité anti-paludique et répondant à la formule générale (VI) : 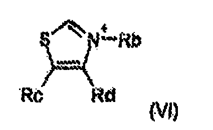 dans laquelle
. Rb représente R₁ ou T, T représentant le groupe de formule : 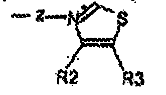 sous réserve que Z ne représente pas un radical alkyle en C6 à C8, lorsque Rcᵢ Rd, R₂ et R₃ représentent un radical méthyle, ou lorsque Rc et Rd d'une part, et R₂ et Rₐ d'autre part, forment ensemble des cycles aromatiques à 6 atomes de carbone,
. Rd représente R₂ ou P, P représentant le groupe de formule : 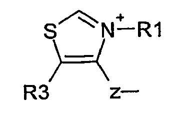
. Rc représente R₃ ou U, U représentant le groupe de formule 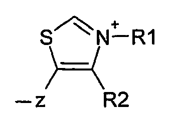
R₁, R₂, R₃ et Z étant tels que définis plus haut,
étant entendu que Rb = T, si Rc = R₃ et Rd = R₂ ; Rd = P, si Rc = R₃ et Rb = R₁ ; et Rc = U, si Rb = R₁, et Rd = R₂.

Selon la présente invention, les composés de formule (VI) peuvent être sous forme de base libre, de sel, d'hydrate ou de solvat.

En particulier, EP 1 196 371 cite parmi les dérivés cyclisés décrits ci-dessus, les dérivés choisis parmi :
- dibromure de 1,12- dodécaméthylène bis [4-méthyl-5-(2-hydroxyéthyl) thiazolium] (T3) ;
- diiodure de 1,12-dodécaméthylène bis[4-méthyl-5-(2-méthoxyéthyl)thiazolium] (T4) ;
- diiodure de 1,12-dodécaméthylènebis(4-méthylthiazolium) (T6) ;
- diiodure de 1,16-hexadécaméthylènebis[4-méthyl-5-(2-méthoxyéthyl)thiazolium] (T8) ;
- diiodure de 3,10-dioxadodécaméthylènebis[5-(1,4-diméthyl)thiazolium] (T9) ;
- dibromure de 3,10-dioxadodécaméthylènebis[5-(1-benzyl,4-méthyl)-thiazolium] (T10) ;
- Diiodure de dodécaméthylènebis[5-(1-méthyl-4-éthoxycarbonyléthyl)-thiazolium] (T12) ;
- Dibromure de dodécaméthylènebis[5-(1-méthyl-4-éthoxycarbonyléthyl)-thiazolium] (T13) ;
- diiodure de dodécaméthylènebis[4-(1-méthyl)-thiazolium] (T15) ;
- dibromure de dodécaméthylènebis[4-(1-benzyl)-thiazolium] (T16).
EP 1 196 371 décrit enfin le procédé d'obtention des sels de bis-thiazolium et de ses précurseurs cités précédemment, notamment la synthèse des composés de formule (VI) tel que T3, T4, T6, T8, T9, T10, T12, T13, T15 et T16. En particulier, la synthèse de T3 est décrite en page 18 de EP 1 196 371. De même, l'activité antipaludique du composé T3 est décrite dans le tableau 13, page 28 de ce même document.

Ainsi, l'invention concerne une association comprenant, en tant que principes actifs, un sel de bis-thiazolium, composé de formule (VI) ou l'un de ses précurseurs, tels que décrit précédemment, et l'artémisinine ou l'un de ses dérivés.

Selon un second objet, l'invention concerne une association entre un sel de bisthiazolium, composé de formule (VI) ou l'un de ses précurseurs tel que décrit précédemment et l'artésunate.

Selon un troisième objet, l'invention concerne une association entre un sel bis-thiazolium, composé de formule (VI), choisi parmi :
- dibromure de 1,12- dodécaméthylène bis [4-méthyl-5-(2-hydroxyéthyl) thiazolium] (T3) ;
- diiodure de 1,12-dodécaméthylène bis[4-méthyl-5-(2-méthoxyéthyl)thiazolium] (T4) ;
- diiodure de 1,12-dodécaméthylènebis(4-méthylthiazolium) (T6) ;
- diiodure de 1, 16-hexadécaméthylènebis[4-méthyl-5-(2-méthoxyéthyl)thiazolium] (T8) ;
- diiodure de 3,10-dioxadodécaméthylènebis[5-(1,4-diméthyl)thiazolium] (T9) ;
- dibromure de 3,10-dioxadodécaméthylènebis[5-(1-benzyl,4-méthyl)-thiazolium] (T10) ;
- Diiodure de dodécaméthylènebis[5-(1-méthyl-4-éthoxycarbonyléthyl)-thiazolium] (T12) ;
- Dibromure de dodécaméthylènebis[5-(1-méthyl-4-éthoxycarbonyléthyl)-thiazolium] (T13) ;
- diiodure de dodécaméthylènebis[4-(1-méthyl)-thiazolium] (T15) ;
- dibromure de dodécaméthylènebis[4-(1-benzyl)-thiazolium] (T16) ;
et l'artésunate.
Selon un quatrième objet, l'invention concerne une association entre un sel de bisthiazolium de formule (VI), le dibromure de 1,12- dodécaméthylène bis [4-méthyl-5-(2-hydroxyéthyl) thiazolium] (T3) et l'artésunate.
Dans ces objets, chacun des principes actifs est utilisé à une concentration reflétant leur puissance respective à guérir l'infection. Dans chacune des associations, les quantités respectives des composés sont fonction de ces doses actives.

Le dibromure de 1,12- dodécaméthylène bis [4-méthyl-5-(2-hydroxyéthyl) thiazolium] (T3) présent dans les associations selon l'invention a la structure qui suit :

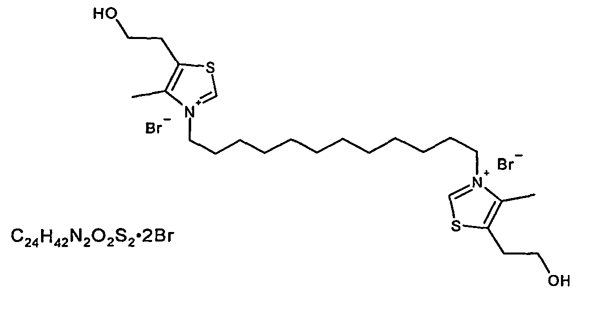

L'artésunate présent dans les associations selon l'invention a pour formule :

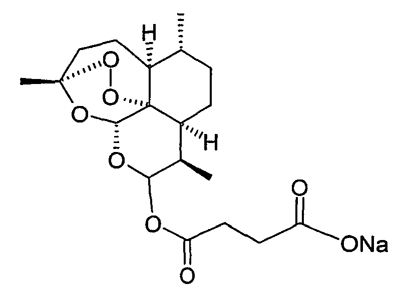

L'invention a encore pour objet une composition pharmaceutique comprenant en tant que principe actif une association entre un sel bis-thiazolium de formule (VI) ou l'un de ses précurseurs, en particulier T3, et l'artémisinine ou l'un de ses dérivés, en particulier l'artésunate, telle que définie précédemment.
Une telle composition pharmaceutique contient des doses thérapeutiquement efficaces d'un sel de bis-thiazolium, composé de formule (VI) ou l'un de ses précurseurs, et d'artémisinine ou de ses dérivés, ainsi qu'au moins un excipient pharmaceutiquement acceptable. Lesdits excipients sont choisis selon la forme pharmaceutique et le mode d'administration souhaité, parmi les excipients habituels qui sont connus de l'Homme du métier. Les fourchettes de proportions de chacun des composés sont définies en fonctions de leur doses exerçant une activité antimalarique en monothérapie et reflètent leur puissance respective à guérir l'infection.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse (bolus ou perfusion), les formes d'administration rectale et les implants. Pour l'application topique, on peut utiliser les composés selon l'invention dans des crèmes, gels, pommades ou lotions.
Des voies d'administration envisagées sont les voies orale, rectale et parentérale, en particulier la voie musculaire, intra rectale et intraveineuse, et notamment la perfusion.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol. Les suspensions peuvent également contenir, au besoin, des adjuvants anti-oxydants, ou les principes actifs selon l'invention peuvent être dilués dans des solutions appropriées telles que du glucose ou du NaCl pour être ultérieurement perfusés.

Par voie parentérale, et en particulier en perfusion, les doses journalières préférées en chacun des deux principes actifs de l'association selon l'invention sont comme indiquées plus haut :
- sel de bis-thiazolium de formule (VI), en particulier T3 : entre 0,01 et 3 mg/kg;
- artémisinine ou l'un de ses dérivés, en particulier artésunate : entre 1 et 5 mg/kg

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

L'association selon l'invention est destinée à être administrée pendant 1 à 4 jours consécutifs, en une ou plusieurs prises quotidiennes de chacun des deux principes actifs, de préférence 1 à 3 prises (orales ou rectales) ou bolus par jour. Ce temps de traitement limité de préférence à 4 jours ou moins est particulièrement avantageux pour faire diminuer de manière massive et rapide la parasitémie et permettre au patient ayant éloigné son pronostique létal d'être ensuite traité contre le paludisme proprement dit. Ce traitement subséquent peut être effectué par une monothérapie par des dérivés d'artémisinine, par tout autre traitement connu du paludisme, mais également par une administration de l'association selon l'invention.
Un exemple de dosage et de mode d'administration peut être de 2,4 mg/kg/jr d'artésunate et 1 mg/kg/jr de T3 durant les premières 24 heures de prise en charge du patient (J1) présentant un paludisme sévère, puis de 1,2 mg/kg/jr d'artésunate et 0,8 mg/kg/jr de T3 durant les 48 heures suivantes (J2 et J3), l'artésunate et le T3 étant administrés par perfusion, dans une poche de perfusion unique à J1 et dans des poches de perfusion séparées à J2 et J3.

L'administration de chacun des deux principes actifs peut être effectuée de façon simultanée, ou bien séparée ou étalée dans le temps (administration séquentielle).
Lorsque l'administration est effectuée de façon simultanée, les deux principes actifs peuvent être réunis au sein d'une forme pharmaceutique unique (combinaison fixe), tel que dans un comprimé ou une gélule adaptés à l'administration orale, ou dans une même poche de perfusion, ou une formulation adaptée à la voie intrarectale.
Les deux principes actifs de l'association selon l'invention peuvent également, que leur administration soit simultanée ou non, être présents dans des formes pharmaceutiques distinctes. A cet effet, les associations selon l'invention peuvent se présenter sous forme de kit comprenant, d'une part, le composé de formule (VI) ou l'un de ses précurseurs, en particulier T3 ou un sel, hydrate ou solvant, et, d'autre part, l'artémisinine ou l'un de ses dérivés, en particulier l'artésunate, ledit composé de formule (VI) tel que T3 et l'artésunate, étant dans des compartiments distincts et étant destinés à être administrés de façon simultanée, séparée ou étalée dans le temps (administration séquentielle).

A titre d'exemple, une forme unitaire d'administration de T3 sous forme de comprimé peut comprendre les composants suivants :

| | |
|---|---|
| T3 | 6 mg |
| Mannitol | 224 mg |
| Croscaramellose sodique | 6 mg |
| Amidon de maïs | 15 mg |
| Hydroxypropyl-méthylcellulose | 2 mg |
| Stéarate de magnésium | 3 mg |

Egalement, à titre d'exemple, une forme unitaire d'administration d'artésunate sous forme de comprimé peut comprendre 50 ou 100 mg d'artésunate et des excipients usuels, par exemple du lactose, du croscarmellose, de la silice colloïdale anhydre, de la cellulose microcristalline et du stéarate de magnesium.
Egalement, à titre d'exemple, une forme unitaire d'administration comprenant une combinaison d'artésunate et de T3 peut comprendre 1,5 mg de T3 et 50 mg d'artésunate, ainsi que des excipients usuels tels que ceux cités ci-dessus, la dose quotidienne pour un adulte étant atteinte en 4 prises d'un comprimé ou en une prise de 4 comprimés.

Les mêmes dosages de T3, d'artésunate, ou de combinaison T3 et artésunate sont utilisés pour les formes unitaires d'administration par voie rectale, avec les excipients connus de l'homme du métier.

Pour un mode d'administration par injection parentérale, une forme unitaire d'administration de T3 peut comprendre 1 mg de T3 dans une ampoule, par exemple, ainsi que des excipients usuels bien connus de l'homme du métier tels que le glycérol et un tampon phosphate, l'ampoule étant prévue pour 10 kg de poids corporel.

Egalement, à titre d'exemple, une forme unitaire d'administration d'artésunate et de T3 dans une ampoule peut comprendre 24 mg d'artésunate et 1 mg de T3, ainsi que des excipients usuels tels que ceux cités ci-dessus, l'ampoule étant administrée à raison d'une ampoule par 10 kg de poids corporel.

La présente invention a également pour objet l'association d'une dose thérapeutique d'au moins un sel de thiazollum, composé de formule (VI) ou l'un de ses précurseurs, en particulier T3, et d'une dose thérapeutique efficace d'artémisinine ou de l'un de ses derivés, en particulier l'artésunate, lesdites doses étant administrées de façon simultanée ou bien séquentielle audit patient, comme cela est décrit précédemment, pour l'utilisation dans le traitement et/ou la prévention du paludisme sévère.

L'invention a encore pour objet une utilisation d'une association comprenant en tant que principes actifs, un sel de bis-thiazolium, composé de formule (VI) ou l'un de ses précurseurs, tels que décrit précédemment et l'artémisinine ou l'un de ses dérivés, en particulier l'artésunate, pour la préparation d'un médicament destiné au traitement ou à la prévention du paludisme sévère.

L'invention a enfin pour objet
- un kit pour l'utilisation dans le traitement ou la prévention du paludisme sévère comprenant, d'une part, au moins un sel de bis-thiazollum, composé de formule (VI) ou l'un de ses précurseurs, et, d'autre part, au moins-l'artémisinine ou l'un de ses dérivés, en particulier l'artésunate, ledit composé de formule (VI) / précurseur et l'artémisinine / dérivé étant dans des compartiments ou poches de perfusion distincts et étant destinés à être administrés de façon simultanée ou séquentielle, et
- un kit tel que défini précédemment, caractérisé en ce qu'il comprend, d'une part, au moins le dibromure de 1,12- dodécaméthylène bis [4-méthyl-5-(2-hydroxyéthyl) thiazolium], et, d'autre part, au moins l'artésunate.

L'association selon l'invention a fait l'objet de tests pharmacologiques et biochimiques *in vivo* chez la souris infectée par un plasmodium de type *Plasmodium falciparum* (souche *Plasmodium vinckei petteri*), permettant de mettre en évidence son efficacité et l'effet synergique qu'elle procure, pour le traitement du paludisme sévère.

### Mesure in vivo de l'activité anti-paludisme chez la souris infectée par Plasmodium vinckei petteri, de l'association du dibromure de 1,12- dodécaméthylène bis [4-méthyl-5-(2-hydroxyéthyl) thiazolium] (T3) avec l'artésunate

### Définitions des termes utilisés dans ce test :

- ED₅₀ = Dose en mg/kg/jour qui entraîne une diminution de 50% de la parasitémie, observée chez les souris infectées,
- ED₉₀ = Dose en mg/kg/jour qui entraîne une diminution de 90% de la parasitémie, observée chez les souris infectées,
- ip = administration intra-péritonéale,
- iv = administration intra-veineuse,
- ir = administration intra-rectale.

### 1. Description du test in vivo utilisé

### -Infection des souris

Au jour 0 (J0), on inocule à des souris femelles de type « Swiss » (OF1, 22-26g), (Charles River Laboratories France, 59 rue de la Paix, 76410 Saint-Aubin-les-Elbeuf), par voie iv dans la veine caudale, 10⁸ érythrocytes parasités suspendus dans 200 µl de NaCl (0,9%). Les parasites utilisés sont de type *Plasmodium vinckei petteri (souche 279 BY, fournie par le Dr. I Landau, Paris, France).*

Les souris sont au préalable, acclimatées pendant deux semaines. Elles sont nourries et boivent *ad libitum.*

L'injection de 10⁸ érythrocytes parasités conduit, au jour 1 (J1) à un taux de parasitémie compris entre 5% et 10% (7,2% ± 0,2%). La souche *Plasmodium vinckei petteri* est maintenue par infection hebdomadaire chez la souris par 10⁷ à 10⁸ érythrocytes parasités suspendus dans un tampon phosphate salin (0,9%)(infection par voie intra-péritonéale).

### - Préparation des solutions de T3 et d'artésunate

Les produits sont dissous dans un tampon phosphate 0,3M (pH = 8,1) La concentration finale de la solution de T3 est comprise entre 40 et 200 mg/l selon la dose, La concentration finale de la solution d'artésunate est comprise entre 100 et 500 mg/l selon la dose
Les produis sont administrés par voie ip. Le volume d'administration est de l'ordre de 100µl, mais dépend du poids de la souris.

### -Traitements

Les souris sont traitées une fois par jour pendant 4 jours aux jours J1, J2, J3 et J4 (Peters, W., J. H. Portus, and B. L. Robinson. 1975. The chemotherapy of rodent malaria. XXII. The value of drug-resistant strains of P. berghei in screening for blood schizonticidal activity. Ann. Trop. Med. Parasitol. 69:155-171 ; and: Ancelin ML, Calas M., Bonhoure A., Herbute S. and Vial H., In vivo antimalarial activity of mono and bis quaternary ammonium salts interfering with Plasmodium phospholipid metabolism. Antimicrob. Agents Chemother., 2003, 47, 2598-2605). Quatre souris sont utilisées par dose.

On administre à l'animal, par voie ip, selon le cas, du T3, de l'artésunate ou du mélange des deux principes actifs solubilisés.

### Mesure de la parasitémie

Au 5^{ème} jour, quelques gouttes de sang sont prélevées à la queue de la souris afin de déterminer la parasitémie par FACS (Fluorescence-activated cell sorter) et de réaliser un frottis sanguin. La parasitémie est d'abord déterminée par FACS sur 20000 cellules. Les globules rouges prélevés pour le FACS sont fixés au glutaraldéhyde puis marqués avec un fluorochrome (YOYO1®) qui marque l'ADN et donc uniquement les cellules parasitées (Barkan, D., Ginsburg, H., and Golenser, J. 2000. Optimisation of flow cytometric measurement of parasitemia in plasmodium-infected mice. Int. J. Parasitol. 30 : 649-653). Les parasitémies inférieures à 15% sont ensuite recomptée sur frottis. Les frottis sont fixés au méthanol puis colorés au Giemsa. On compte au microscope le nombre de cellules sanguines parasitées. La parasitémie est exprimée en pourcentage d'érythrocytes infectés présents dans le prélèvement sur un échantillon de 2000 cellules. Les ED₅₀ et ED₉₀ sont déterminées à J5. La parasitémie est déterminée du 1^{er} au 10^{ième} jour de traitement, puis aux 15^{ième}, 22^{ième} et 47^{ième} jours.
Les souris pour lesquelles le frottis à J5 ne révèle aucune trace de parasites seront contrôlées à nouveau pendant au moins 1 mois après la fin du traitement, afin de détecter une possible recrudescence des parasites. La dose curative est la dose en produit qui assure la survie de l'ensemble du lot d'animaux traité après un mois

### Détermination des ED₅₀

Le 0% d'inhibition correspond à la moyenne des parasitémies observées chez les souris infectées non traitées. Le 100% d'inhibition correspond à une parasitémie très faible ou nulle, inférieure à 0,01 %. Les ED₅₀ sont déterminés par interpolation linéaire de la courbe dose réponse représentée en logarithme des concentrations.

### 2. Mesure de l'activité anti-paludique du T3 et de l'artésunate sur une souche de parasite Plasmodium vinckei petteri in vivo

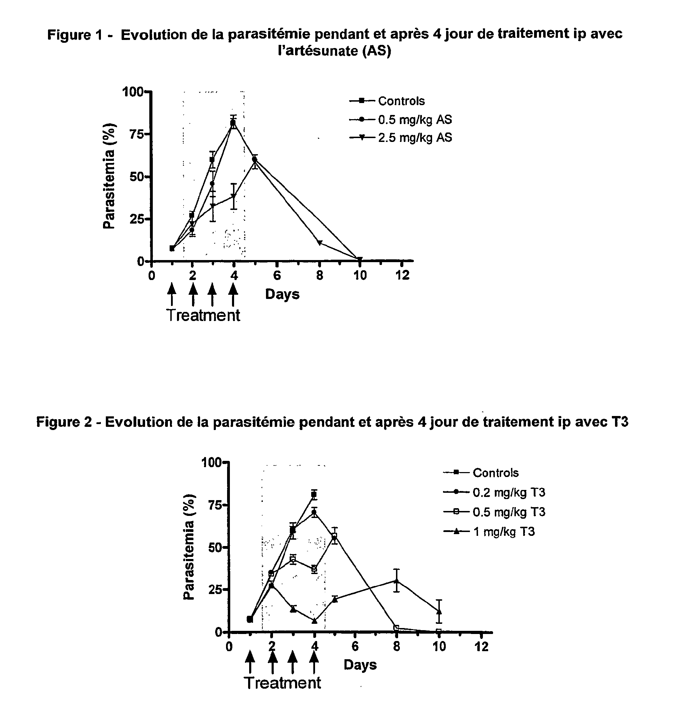

Afin de mettre en évidence un effet synergique entre les deux composés il est important de connaitre leur activité lorsqu'ils sont administrés seuls, afin de les associer à des doses proches de leur ED50. Le cas de leur administration par voie parentérale correspond à la situation clinique du paludisme sévère. Dans ce cas il est important de faire chuter rapidement la parasitémie au cours des premiers jours de traitement. Aux doses testées l'artésunate administré seul ne fait pas chuter la parasitémie le premier jour ainsi que les jour suivants (figure 1). T3 fait chuter efficacement la parasitémie après 4 jours de traitement a la dose de 1mg/kg/j mais n'empêche pas une montée de la parasitémie après le premier jour de traitement (figure2).
Les ED₅₀ obtenues sont présentées dans le tableau I qui suit :

**Tableau I**

| | ED₅₀ (mg/kg/jour) |
|---|---|
| T3 | 0,55 |
| artésunate | > 2,5 |

Plus l'ED₅₀ est faible, meilleure est l'activité. Ainsi, T3 seul montre une meilleure activité sur la parasitémie (0,55 mg/kg/jour) que l'artésunate seul (>2,5 mg/kg/jour). De plus T3 seul, à une dose de 0,5 mg/kg/jour, permet une guérison totale après un mois (résultats non montrés) alors que l'artésunate seul, à une dose de 2,5 mg/kg/jour, ne le permet pas.

### 3. Mesure de l'activité anti-paludisme de l'association T3/artésunate sur une souche de parasite Plasmodium vinckei petteri in vivo

Pour cette étude en association, on a choisi la dose de 2,5 mg/kg/j d'artésunate (légèrement inférieure mais proche de L'ED 50), la dose de 0,5 mg/kg/j étant totalement inactive sur la parasitémie à 4 jours. T3 a été évalué en association à une dose inferieure, proche et supérieure à l'ED₅₀, soit 0,2, 0,5 et 1 mg/kg/j

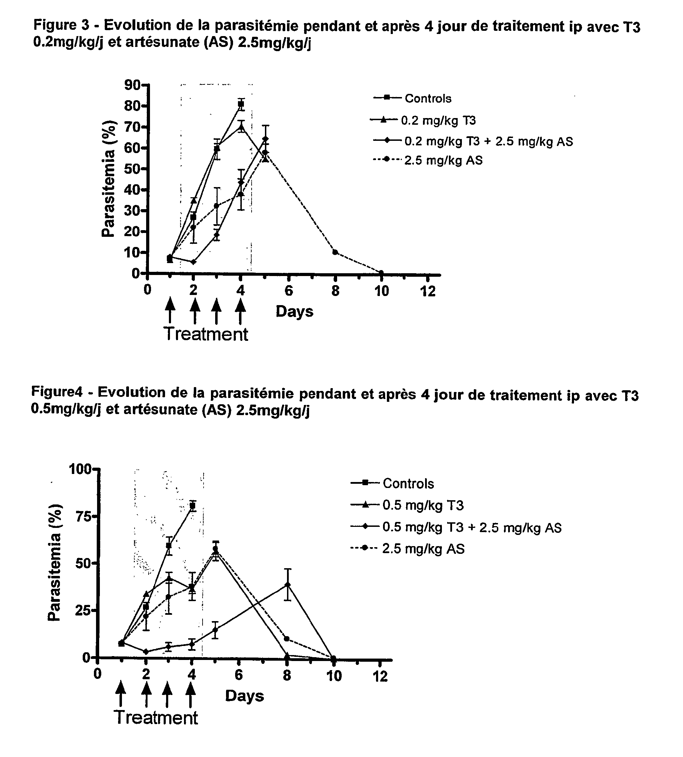

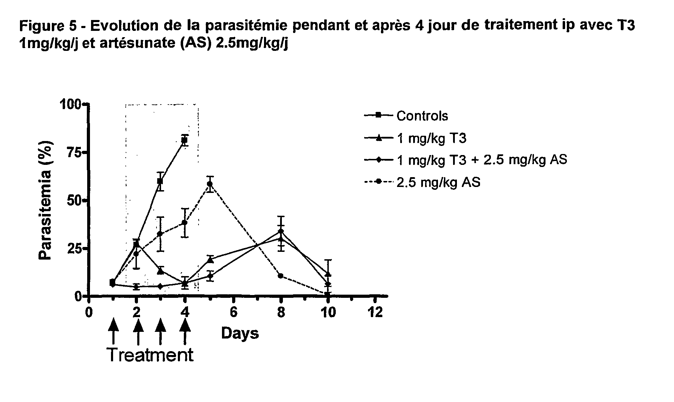

De façon surprenante, comme indiqué dans les figures 3, 4 et 5 l'association comprenant T3 (à toutes les doses précitées) avec de l'artésunate présent à une dose de 2,5 mg/kg/jour, permet de diminuer significativement le taux de parasitémie, par comparaison avec l'administration séparée de T3 seul ou d'artésunate seul. Notamment, il est important de constater que 24 h après la première administration (jour 2) et quelque soit la dose de T3 administrée on observe une diminution de la parasitémie des souris alors qu'à ce temps les produits administrés individuellement sont inactifs. Le tableau II (et figures 3,4,5) fournit la moyenne des parasitémies relevées à ce jour 2 (4 animaux par dose). Il est à noter qu'il existe un effet statistiquement synergique 24 heures après la première administration (jour 2) pour le T3 à des doses de 0,2, 0,5 et 1 mg/kg en association avec de l'artésunate à une dose de 2,5 mg/kg. Cet effet synergique très significatif après le premier jour de traitement est particulièrement important dans le cas du traitement du paludisme sévère, où le clinicien recherche justement une réduction massive et rapide de la parasitémie du patient afin d'éloigner le pronostique mortel.

**Tableau II**

| Artesunate | T3 | parasitemie J1 | parasitemie J2 | parasitemie J5 |
|---|---|---|---|---|
| (mg/kg/j) | (mg/kg/j) | | | |
| 0 | 0 | 7,20% | 26,90% | death |
| 2,5 | 0 | 7,00% | 32,90% | 58,20% |
| 2,5 | 0,2 | 8,10% | 5,70% | 54,90% |
| 2,5 | 0,5 | 8,30% | 3,40% | 15,10% |
| 2,5 | 1 | 6,10% | 5,10% | 10,70% |
| 0 | 0,2 | 6,60% | 35,00% | 55,20% |
| 0 | 0,5 | 7,80% | 34,00% | 56,80% |
| 0 | 1 | 7,20% | 27,90% | 19,40% |

A partir de la dose en T3 de 0,5 mg/kg/jr associée à l'artésunate 2.5mg/kg/j la parasitémie est maintenue à sa valeur initiale avant traitement pendant toute la durée du traitement alors que les administrations individuelles en produits ne le permettent pas (tableau II, figures 3,4,5).
Le composé T3 est responsable de l'effet majoritaire sur la cure totale des souris après un mois. La co-administration de l'artésunate à 2.5 mg/kg/jr n'améliore pas la dose curative obtenue avec T3 seul.

Le tableau III résume les résultats discutés ci-dessus.

**Tableau III**

| | | | | T3 (mg/kg/j) |
|---|---|---|---|---|
| | | 0 | 0,2 | 0,5 1 |
| Artesunate (mg/kg/j) | 0 | | réduction de la croissance parasitaire dose dépendante de J3 à J5 | réduction de la croissance parasitaire dose dépendante de J3 à J5, dose curative |
| | 2,5 | réduction de la croissance parasitaire dose dépendante de J3 à J5 | réduction de la parasitemie par rapport à J0, à J1 | réduction de la parasitemie par rapport a J0 pendant toute la duree du traitement |

Ainsi, lorsque les 2 principes actifs sont administrés en combinaison, la parasitémie diminue de façon significative pendant les 4 premiers jours de traitement. L'effet est très nettement supérieur à des doses comparables, voire plus faibles que celles déterminées pour l'administration de chacun des deux principes actifs pris individuellement.

Notamment lorsque T3 est administré en association avec l'artésunate, l'artésunate étant présent à une dose d'au moins 2,5 mg/kg/jour, un effet bénéfique est observé sur la diminution de la parasitémie. On comprend donc que l'association des deux principes actifs est particulièrement efficace dans une fenêtre définie par la dose minimale de l'un des composés et le ratio de l'un des composés par rapport à l'autre, à l'intérieur de cette fenêtre.

Les résultats obtenus *in vivo* chez la souris infectée par *P. vinckei petteri* prouvent que l'association selon l'invention, de l'artémisinine ou l'un de ses dérivés (en particulier l'artésunate) et d'un sel de bis-thiazolium, composé de formule (VI), en particulier T3 est avantageuse pour le traitement du paludisme sévère.

## Revendications

1. Association, comprenant en tant que principes actifs
▩un sel de bis-thiazolium, composé de formule (VI) : 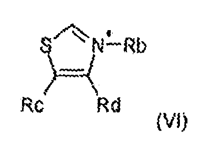 dans laquelle
. Rb représente :
- un groupe R₁ représentant lui-même un atome d'hydrogène, un radical alkyle en C1 à C5, éventuellement substitué par un radical aryle (notammant un radical phényle), un hydroxy, un alkoxy, dans lequel le radical alklyle comprend de 1 à 5 atomes de carbone, ou aryloxy (notamment phénoxy),
- ou bien un groupe T, T représentant le groupe de formule : 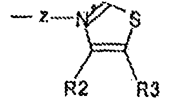
dans lequel :
- R₂ représente un atome d'hydrogène, un radical alkyle en C1 à C5, ou un groupe -CH₂-COO-alkyl(C1 à C5) ;
- R₃ représente un atome d'hydrogène, un radical alkyle ou alkényle en C1 à C5, le cas échéant substitué par -OH, un groupement phosphate, un radical alkoxy, dans lequel le radical alkyle est en C1 à C3, ou aryloxy; ou un groupe alkyl (ou aryl)carbonyloxy ;
ou encore R₂ et R₃ forment ensemble un cycle à 5 ou 6 atomes de carbone ; R₂ et R₃ peuvent être reliés pour former un cycle ou un hétérocycle de 5 à 7 chaînons comprenant éventuellement un ou plusieurs hétéroatomes O et/ou S.
- Z représente un radical alkyle en C6 à C21, notamment de C12 ou C13 à C21 le cas échéant avec insertion d'une ou de plusieurs liaisons multiples, et/ou d'un ou plusieurs hétéroatomes O et/ou S, et/ou d'un ou de plusieurs cycles aromatiques, et les sels pharmaceutiquement acceptables de ces composés,
sous réserve que Z ne représente pas un radical alkyle en C6 à C8, lorsque Rc, Rd, R₂ et R₃ représentent un radical méthyle, ou lorsque Rc et Rd d'une part, et R₂ et R₃ d'autre part, forment ensemble des cycles aromatiques à 6 atomes de carbone,
. Rd représente R₂ tel que précédemment défini ou P, P représentant le groupe de formule : 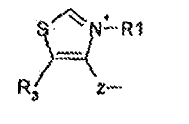
. Rc représente R₃ tel que précédemment défini ou U, U représentant le groupe de formule : 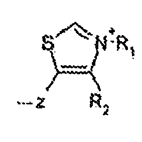 étant entendu que Rb = T, si Rc = R₃ et Rd = R₂ ; Rd = P, si Rc = R₃ et Rb = R₁ ; et Rc = U, si Rb = R₁, et Rd = R₂, sous forme de base libre, de sel, d'hydrate ou de solvat,
▩ou l'un de ses précurseurs choisi parmi les composés de formule (I) à (V),
▩▩ le composé (I) répondant à la formule : 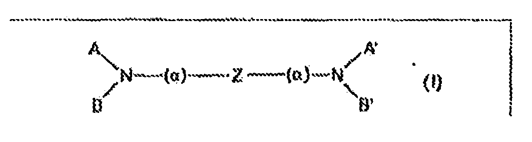 dans laquelle
• A et A' sont identiques ou différents l'un de l'autre et représentent :
. soit, respectivement, un groupe A, et A'₁ de formule : 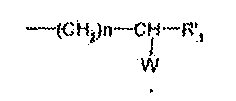
où n est un entier de 2 à 4 : R'₁ représente un atome d'hydrogène, un radical alkyle en C1 à C5, éventuellement substitué par un radical aryle (notamment un radical phényle), un hydroxy, un alkoxy, dans lequel le radical alkyle comprend de 1 à 5 atomes de carbone, ou Aryloxy (notamment phénoxy) :
et W représente un atome d'halogène choisi parmi le chlore, le brome ou l'iode, ou un groupe nucléofuge, comme le radical tosyle CH₃-C₆H₄-SO₃, mésityle CH₃-SO₃, CF₃-SO₃. NO₂-C₆H₄-SO₃,
soit un groupe A₂ qui représente un radical formyle -CHO, ou acétyle -CO-CH₃,
- B et B' sont identiques ou différents l'un de l'autre et représentent :
soit respectivement les groupes B₁ et B'₁, si A et A' représentent respectivement A₁ et A'₁, B₁ et B'₁ représentant un groupe R₁ qui présente la même définition que R'₁ ci-dessus, mais ne peut pas être un atome d'hydrogène.
soit respectivement les groupes B₂ et B'₂, si A et A' représentent A₂, B₂ ou B'₂ étant le groupe R₁ tel que défini ci-dessus, ou un groupement de formule : 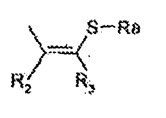
dans lequel -Ra représente un groupe RS- ou RCO-, ou R est un radical alkyle en C1 à C6, notamment de C1 à C5, linéaire, ramifié, ou cyclique, le cas échéant substitué par un ou plusieurs groupes hydroxy, alcoxy ou aryloxy, ou un groupe amino et/ou un groupe - COOH ou COOM, ou M est un alkyle en C1 à C3 ; un radical phényle ou benzyle, dans lequel le radical phényle est le cas échéant substitué par au moins un radical alkyle ou alcoxy en C1 à C5, ceux-ci étant éventuellement substitués par un groupe amino, ou par un hétérocycle azoté ou oxygéné, un groupe -COOH ou -COOM ; ou un groupe -CH₂-hétérocycle, à 5 ou 6 éléments, azoté et/ou oxygéné;
R₂ représente un atome d'hydrogène, un radical alkyle en C1 à C5, ou un groupe -CH₂-COO-alkyl (C1 à C5);
et R₃ représente un atome d'hydrogène, un radical alkyle ou alkènyle en C1 à C5, le cas échéant substitué par -OH, un groupement phosphate, un radical alkoxy, dans lequel le radical alkyle est en C1 à C3, ou aryloxy; ou un groupe alkyl (ou aryl)carbonyloxy ;
ou encore R₂ et R₃ forment ensemble un cycle à 5 ou 6 atomes de carbone ; R et R₃ peuvent être reliés pour former un cycle à 5 à 7 atomes (carbone, oxygéne, soufre)
- o représente ;
soit une simple liaison, lorsque A et A' représentent A1 et A'1 ; ou lorsque A et A' représentent A₂, c'est-à-dire un groupe -CHO ou -COCH₃, et B₂ et B'₂ représentent : 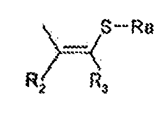
soit, lorsque A et A' représentent A₂ et B₂ et B'₂ représentent R₁, un groupement de formule: 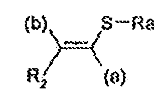 ou un groupement de formule : 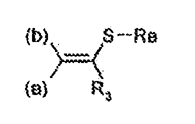 dans lesquels (a) représente une liaison vers Z et (b) une liaison vers l'atome d'azote,
- Z représente un radical alkyle en C6 à C21, notamment de C12 ou C13 à C21, le cas échéant avec insertion d'une ou de plusieurs liaisons multiples, et/ou d'un ou plusieurs hétéroatomes O et/ou S, et/ou d'un ou de plusieurs cycles aromatiques, et les sels pharmaceutiquement acceptables de ces composés, sous réserve que R'₁ ne représente pas H ou un radical alkyl en C1 ou C2, lorsque n = 3 ou 4, R₁ représente un radical alkyle en C1 à C4 et Z représente un radical alkyle en C6 à C10 ;
■■ le composé (II) répondant à la formule: 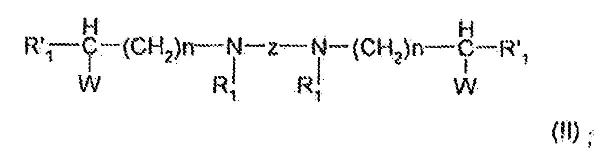 ■■ le composé (III) répondant à la formule : 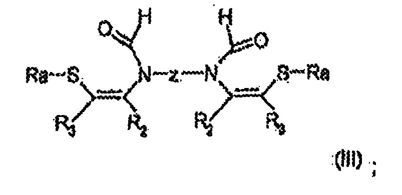 ■■ le composé (IV) répondant à la formule: 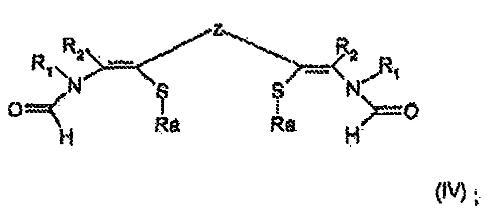 ■■ le composé (V) répondant à la formule : 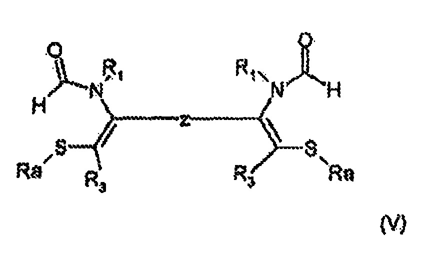
dans lesquelles Rₐ, R₁, R'₁, R₂, R₃, W, n et Z sont tels que définis précédemmemt,
et
■ l'artémisinine ou l'un de ses dérivés choisi parmi l'artésunate, l'artémétiner, l'artééther, l'hydroartémisinine ou la dihydroartémisinine,
pour l'utilisation dans le traitement et/ou la prévention du paludisme sévère.

2. Association pour utilisation selon revendication 1, **caractérisés en ce que** le sel bis-thiezolium, composé de formule (VI) est choisi parmi:
- dibromure de 1,12- dodécaméthylène bis [4-méthyl-6-(2-hydroxyéthyl) thiézolium]:
- dilodure de 1,12-dodécaméthyléne bis[4-méthyl-5-(2-méthoxyéthyl)thiazolium];
- dilodure de 1,12-dodécaméthylénable(4-méthylthiazolium) ;
- dilodure de 1,16-hexadécaméthyléneble[4-méthyl-5-(2-méthoxyéthyl)thiazol];
- diiodure de 3,10-dioxadodécaméthylénebis[5-(1,4-diméthyl)thiazolium];
- dibromure de 3,10-dioxadodécaméthylénebis[5-(1-benzl,4-méthyl)-thiaxolium] ;
- Diiodure de dodécaméthylènebis[5-(1-méthyl-4-éthoxycarbonyléthyl)-thiazolium] ;
- Dibromure de dodécaméthylènebis[5-(1-méthyl-4-éthoxycarbonyléthyl)-thiazolium] ;
- Diiodure de dodécaméthylènebis[4-(1-méthyl)-thiazolium] :
- dibromure de dodécaméthylènebis[4-(1-benzyl)-thiazolium].

3. Association pour utilisation selon l'une quelconque des revendications 1 à 2, **caractérisée en ce que** chacun des principes actifs est destiné à être administré de façon simultanée ou séquentielle.

4. Composition pharmaceutique comprenant des doses thérapeutiquement efficaces d'artémisinine ou de l'un de ses dérivés, et d'au moins un sel de bis-thiazolium, composé de formule (VI), ou l'un de ses précurseurs, tels que définis dans la revendication 1, ainsi qu'au moins un excipient pharmaceutiquement acceptable, pour l'utilisation dans le traitement et/ou la prévention du paludisme sévère.

5. Composition pharmaceutique pour utilisation selon la revendication 4, **caractérisée en ce que** le sel de bis-thiazolium, composé de formule (VI) est le dibromure de 1,12-dodécaméthylène bis [4-méthyl-5-(2-hydroxyéthyl) thiazolium].

6. Composition pharmaceutique pour utilisation selon la revendication 4 ou la revendication 5 **caractérisée en ce que** le dérivé d'artémisinine est l'artésunate.

7. Composition pharmaceutique pour utilisation selon l'une quelconque des revendications 4 à 6, **caractérisée en ce qu'**elle est adaptée à l'administration orale, rectale ou injectable, et de préférence adaptée à l'injection intraveineuse, particulièrement sous forme de perfusion.

8. Utilisation d'une association selon l'une quelconque des revendications 1 à 3, pour la préparation d'un médicament destiné au traitement ou à la prévention du paludisme sévère.

9. Kit pour l'utilisation dans le traitement ou la prévention du paludisme sévère comprenant, d'une part, au moins un sel de bis-thiazopilum, composé de formule (VI) ou l'un de ses précurseurs, et, d'autre part, au moins l'artèmisinine ou l'un de ses dérivés, tels que définis dans la revendication 1, ledit composé de formule (VI) ou l'un de ses précurseurs et l'artémisinine ou l'un de ses dérivées étant dans des compartiments distincts et étant destinés à être administrés de façon simultanée ou séquentielle.

10. Kit pour utilisation selon la revendication 10, **caractérisé en ce qu'**il comprend, d'une part, au moins le dibromure de 1,12- dodécaméthyléne bis [4-méthyl-5-(2-hydroxyéthyl) thiazolium], et d'autre part, au moins l'artésunate.

## Claims

1. Combination comprising, as active ingredients,
■ a bisthiazolium salt, which is a compound of formula (VI) : 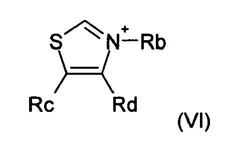 in which
. Rb represents:
- a group R₁ which itself represents a hydrogen atom, a C₁ to C₅ alkyl radical, optionally substituted with an aryl radical (in particular a phenyl radical), a hydroxyl, an alkoxy, in which the alkyl radical contains from 1 to 5 carbon atoms, or aryloxy (in particular phenoxy),
- or else a group T, T representing the group of formula: 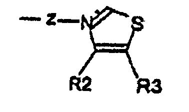
in which:
- R₂ represents a hydrogen atom, a C₁ to C₅ alkyl group or a -CH₂-COO- (C₁ to C₅) alkyl group;
- R₃ represents a hydrogen atom, a C₁ to C₅ alkyl or alkenyl radical, as appropriate substituted with -OH, a phosphate group, an alkoxy radical, in which the alkyl radical is C₁ to C₃, or aryloxy; or an alkyl (or aryl)carbonyloxy group;
or else R₂ and R₃ together form a ring containing 5 or 6 carbon atoms; R₂ and R₃ may be linked so as to form a ring or a heterocycle containing 5 to 7 members, optionally comprising one or more heteroatoms O and/or S;
- Z represents a C₆ to C₂₁, in particular C₁₂ or C₁₃ to C₂₁, alkyl radical, as appropriate with insertion of one or more multiple bonds, and/or one or more heteroatoms O and/or S, and/or of one or more aromatic rings, and the pharmaceutically acceptable salts of these compounds,
with the proviso that Z does not represent a C₆ to C₈ alkyl radical, when Rc, Rd, R₂ and R₃ represent a methyl radical, or when Rc and Rd, on the one hand, and R₂ and R₃, on the other hand, together form aromatic rings containing 6 carbon atoms;
. Rd represents R₂ as defined above or P, P representing the group of formula: 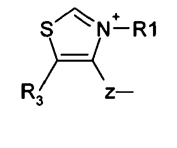
. Rc represents R₃ as defined above or U, U representing the group of formula: 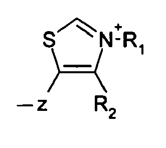 it being understood that Rb = T, if Rc = R₃ and Rd = R₂; Rd = P, if Rc = R₃ and Rb = R₁; and Rc = U, if Rb = R₁, and Rd = R₂, in the form of a free base, of a salt, of a hydrate or of a solvate,
■ or a precursor thereof chosen from the compounds of formulae (I) to (V),
■■ the compound (I) corresponding to the formula: 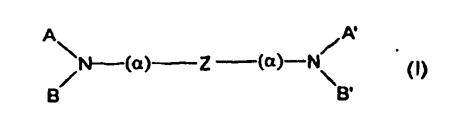 in which
- A and A' are identical to or different from one another and represent:
. either, respectively, a group A₁ and A'₁ of formula: 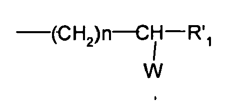 where n is an integer from 2 to 4; R'₁ represents a hydrogen atom, a C₁ to C₅ alkyl radical, optionally substituted with an aryl radical (in particular a phenyl radical), a hydroxyl, an alkoxy, in which the alkyl radical contains from 1 to 5 carbon atoms, or aryloxy (in particular phenoxy);
and W represents a halogen atom chosen from chlorine, bromine or iodine, or a nucleofugal group, such as the tosyl radical CH₃-C₆H₄-SO₃, the mesityl radical CH₃-SO₃, the CF₃-SO₃ radical or the NO₂-C₆H₄-SO₃ radical,
. or a group A₂ which represents a formyl radical -CHO or an acetyl radical -CO-CH₃,
- B and B' are identical to or different from one another and represent:
. either, respectively, the groups B₁ and B'₁, if A and A' represent, respectively, A₁ and A'₁, B₁ and B'₁ representing a group R₁ which has the same definition as R'₁ above, but cannot be a hydrogen atom,
. or, respectively, the groups B₂ and B'₂, if A and A' represent A₂, B₂ or B'₂ being the group R₁ as defined above, or a group of formula: 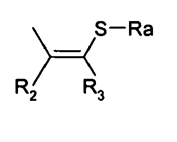
in which -Ra represents an RS- or RCO- group, where R is a linear, branched or cyclic, C₁ to C₆, in particular C₁ to C₅, alkyl radical, as appropriate substituted with one or more hydroxyl, alkoxy or aryloxy groups, or an amino group and/or a -COOH or COOM group, where M is a C₁ to C₃ alkyl; a phenyl or benzyl radical, in which the phenyl radical is, as appropriate, substituted with at least one C₁ to C₅ alkyl or alkoxy radical, said radicals being optionally substituted with an amino group, or with a nitrogenous or oxygenated heterocycle, a -COOH group or a -COOM group; or a group -CH₂-heterocycle, in which the heterocycle has 5 or 6 elements and is nitrogenous and/or oxygenated;
R₂ represents a hydrogen atom, a C₁ to C₅ alkyl radical or a -CH₂-COO (C₁ to C₅) alkyl group;
and R₃ represents a hydrogen atom; a C₁ to C₅ alkyl or alkenyl radical, as appropriate substituted with -OH, a phosphate group, an alkoxy radical, in which the alkyl radical is C₁ to C₃, or aryloxy; or an alkyl (or aryl)carbonyloxy group;
or else R₂ and R₃ together form a ring containing 5 or 6 carbon atoms; R and R₃ may be linked so as to form a ring containing 5- to 7 atoms (carbon, oxygen, sulphur);
- a represents:
. either a single bond, when A and A' represent A1 and A'1: or when A and A' represent A₂, i.e. a -CHO or -COCH₃ group, and B₂ and B'₂ represent: 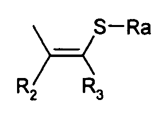
. or, when A and A' represent A₂ and B₂ and B'₂ represent R₁, a group of formula: 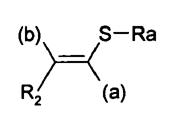 or a group of formula: 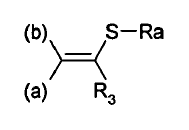 in which (a) represents a bond towards Z and (b) a bond towards the nitrogen atom,
- Z represents a C₆ to C₂₁, in particular C₁₂ or C₁₃ to C₂₁, alkyl radical, as appropriate with insertion of one or more multiple bonds, and/or one or more heteroatoms O and/or S, and/or one or more aromatic rings, and the pharmaceutically acceptable salts of these compounds, with the proviso that R'₁ does not represent H or a C₁ or C₂ alkyl radical, when n = 3 or 4, R₁ represents a C₁ to C₄ alkyl radical and Z represents a C₆ to C₁₀ alkyl radical;
■■ the compound (II) corresponding to the formula: 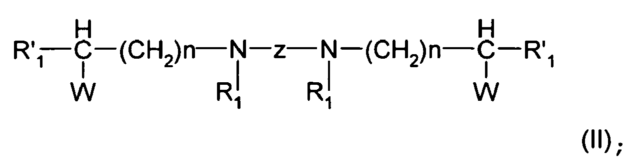 ■■ the compound (III) corresponding to the formula: 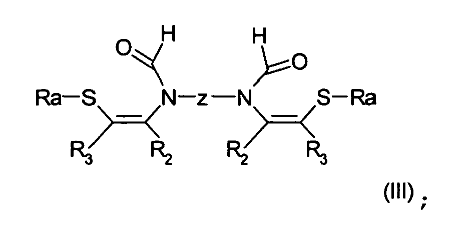 ■■ the compound (IV) corresponding to the formula: 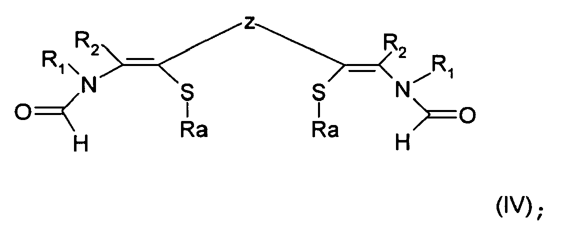 ■■ the compound (V) corresponding to the formula: 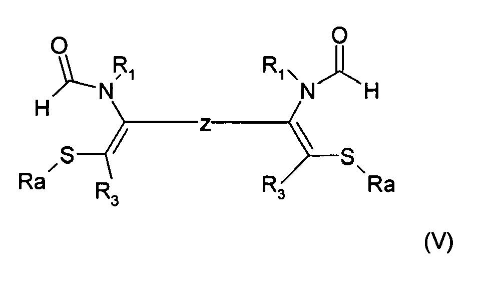 in which Ra, R₁, R'₁, R₂, R₃, W, n and Z are as defined above,
and
■ artemisinin or a derivative thereof, chosen from artesunate, artemether, arteether, hydroartemisinin or dihydroartemisinin,
for use in the treatment and/or prevention of severe malaria.

2. Combination for use according to Claim 1, **characterized in that** the bisthiazolium salt, which is a compound of formula (VI), is chosen from:
- 1,12-dodecamethylenebis[4-methyl-5-(2-hydroxyethyl)thiazolium] dibromide;
- 1,12-dodecamethylenebis[4-methyl-5-(2-methoxyethyl)thiazolium] diiodide;
- 1,12-dodecamethylenebis(4-methylthiazolium) diiodide;
- 1,16-hexadecamethylenebis[4-methyl-5-(2-methoxyethyl)thiazolium] diiodide;
- 3,10-dioxadodecamethylenebis[5-(1,4-dimethyl)thiazolium] diiodide;
- 3,10-dioxadodecamethylenebis[5-(1-benzyl-4-methyl)thiazolium] dibromide;
- dodecamethylenebis[5-(1-methyl-4-ethoxycarbonylethyl)thiazolium] diiodide;
- dodecamethylenebis[5-(1-methyl-4-ethoxycarbonylethyl)thiazolium] dibromide;
- dodecamethylenebis[4-(1-methyl)thiazolium] diiodide;
- dodecamethylenebis[4-(1-benzyl)thiazolium] dibromide.

3. Combination for use according to either one of Claims 1 and 2, **characterized in that** each of the active ingredients is intended to be administered simultaneously or sequentially.

4. Pharmaceutical composition comprising therapeutically effective doses of artemisinin or of a derivative thereof, and of at least one bisthiazolium salt, which is a compound of formula (VI), or a precursor thereof, as defined in Claim 1, and also at least one pharmaceutically acceptable excipient, for use in the treatment and/or prevention of severe malaria.

5. Pharmaceutical composition for use according to Claim 4, **characterized in that** the bisthiazolium salt, which is a compound of formula (VI), is 1,12-dodecamethylenebis[4-methyl-5-(2-hydroxyethyl)thiazolium] dibromide.

6. Pharmaceutical composition for use according to Claim 4 or Claim 5, **characterized in that** the artemisinin derivative is artesunate.

7. Pharmaceutical composition for use according to any one of Claims 4 to 6, **characterized in that** it is suitable for oral, rectal or injectable administration, and preferably suitable for intravenous injection, particularly in the form of a drip.

8. Use of a combination according to any one of Claims 1 to 3, for the preparation of a medicament for use in the treatment or prevention of severe malaria.

9. Kit for use in the treatment or prevention of severe malaria, comprising, on the one hand, at least one bisthiazolium salt, which is a compound of formula (VI) or a precursor thereof, and, on the other hand, at least artemisinin or a derivative thereof, as defined in Claim 1, said compound of formula (VI) or a precursor thereof and the artemisinin or a derivative thereof being in distinct compartments and being intended to be administered simultaneously or sequentially.

10. Kit for use according to Claim 10, **characterized in that** it comprises, on the one hand, at least 1,12-dodecamethylenebis[4-methyl-5-(2-hydroxyethyl)thiazolium] dibromide, and, on the other hand, at least artesunate.

## Patentansprüche

1. Kombination, umfassend als Wirkstoffe
■ ein Bisthiazoliumsalz der Formel (VI): 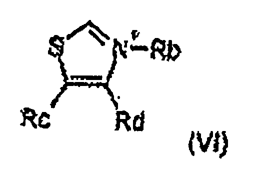 worin
. Rb für:
- eine Gruppe R₁, die selbst für ein Wasserstoffatom, einen C₁- bis C₅-Alkylrest, der gegebenenfalls durch einen Arylrest (insbesondere einen Phenylrest), ein Hydroxy, ein Alkoxy, worin der Alkylrest 1 bis 5 Kohlenstoffatome umfaßt, oder Aryloxy (insbesondere Phenoxy) substituiert ist,
- oder auch eine Gruppe T steht, wobei T für die Gruppe der Formel: 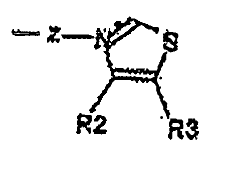
steht, worin:
- R₂ für ein Wasserstoffatom, einen C₁- bis C₅-Alkylrest oder eine -CH₂-COO- (C₁-C₅) -Alkylgruppe steht;
- R₃ für ein Wasserstoffatom, einen C₁- bis C₅-Alkyl- oder Alkenylrest, der gegebenenfalls durch -OH, eine Phosphatgruppe, einen Alkoxyrest, worin der Alkylrest 1 bis 3 Kohlenstoffatome umfaßt, oder Aryloxy substituiert ist; oder eine Alkylcarbonyloxygruppe (oder Arylcarbonyloxygruppe) steht;
oder auch R₂ und R₃ zusammen einen Ring mit 5 oder 6 Kohlenstoffatomen bilden; wobei R₂ und R₃ unter Bildung eines 5- bis 7-gliedrigen Heterocyclus, der gegebenenfalls ein oder mehrere O- und/oder S-Heteroatome umfaßt, miteinander verbunden sein können;
- Z für einen C₆- bis C₂₁-Alkylrest, insbesondere C₁₂- oder C₁₃- bis C₂₁-Alkylrest steht, gegebenenfalls mit Einschub einer oder mehrerer Mehrfachbindungen und/oder eines oder mehrerer O- und/oder S-Heteroatome und/oder eines oder mehrerer aromatischer Ringe, und die pharmazeutisch unbedenklichen Salze dieser Verbindungen,
mit der Maßgabe, daß Z nicht für einen C₆- bis C₈-Alkylrest steht, wenn Rc, Rd, R₂ und R₃ für einen Methylrest stehen oder wenn Rc und Rd einerseits und R₂ und R₃ andererseits zusammen aromatische Ringe mit 6 Kohlenstoffatomen bilden;
. Rd für R₂ gemäß obiger Definition oder P steht, wobei P für die Gruppe der Formel: 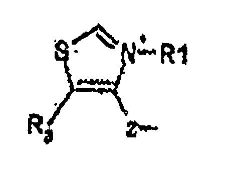
steht;
. Rc für R₃ gemäß obiger Definition oder U steht, wobei U für die Gruppe der Formel: 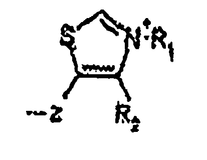 steht;
wobei es sich versteht, daß Rb = T, wenn Rc = R₃ und Rd = R₂; Rd = P, wenn Rc = R₃ und Rb = R₁; und Rc = U, wenn Rb = R₁ und Rd = R₂, in Form einer freien Base, eines Salzes, eines Hydrats oder eines Solvats,
■ oder eine Vorstufe davon, ausgewählt unter den Verbindungen der Formel (I) bis (V),
■■ wobei die Verbindung (I) der Formel: 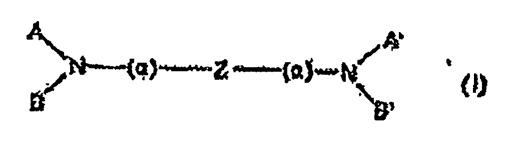 entspricht, worin
- A und A' gleich oder voneinander verschieden sind und für:
. entweder eine Gruppe A₁ bzw. A'₁ der Formel: 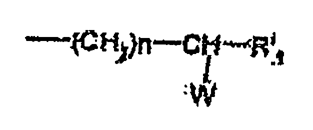
worin n für eine ganze Zahl von 2 bis 4 steht; R'₁ für ein Wasserstoffatom oder einen C₁- bis C₅-Alkylrest, der gegebenenfalls durch einen Arylrest (insbesondere einen Phenylrest), ein Hydroxy, ein Alkoxy, worin der Alkylrest 1 bis 5 Kohlenstoffatome umfaßt, oder Aryloxy (insbesondere Phenoxy) substituiert ist, steht
und W für ein unter Chlor, Brom oder Iod ausgewähltes Halogenatom oder eine nucleofuge Gruppe wie den Tosylrest CH₃-C₆H₄-SO₃, den Mesitylrest CH₃-SO₃, den CF₃-SO₃-Rest oder den NO₂-C₆H₄-SO₃-Rest steht,
. oder eine Gruppe A₂, die für einen Formylrest -CHO oder einen Acetylrest -CO-CH₃ steht, stehen,
- B und B' gleich oder voneinander verschieden sind und für:
. entweder die Gruppen B₁ bzw. B'₁ stehen, wenn A und A' für A₁ bzw. A'₁ stehen, wobei B₁ und B'₁ für eine Gruppe R₁, die die gleiche Definition wie R'₁ aufweist, aber nicht für ein Wasserstoffatom stehen kann, stehen,
. oder die Gruppen B₂ bwz. B'₂ stehen, wenn A und A' für A₂ stehen, wobei B₂ oder B'₂ für die Gruppe R₁ gemäß obiger Definition oder eine Gruppe der Formel: 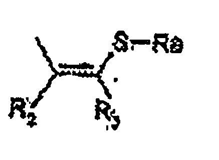 steht, worin -Ra für eine RS- oder RCO-Gruppe, wobei R für einen linearen, verzweigten oder cyclischen C₁- bis C₆-Alkylrest, insbesondere C₁-bis C₅-Alkylrest, steht, der gegebenenfalls durch eine oder mehrere Hydroxy-, Alkoxy- oder Aryloxygruppen oder eine Aminogruppe und/oder eine -COOH-oder COOH-Gruppe substituiert ist, wobei M für ein C₁- bis C₃-Alkyl steht; einen Phenyl- oder Benzylrest, worin der Phenylrest gegebenenfalls durch mindestens einen C₁- bis C₅-Alkyl- oder -Alkoxyrest, wobei diese Reste gegebenenfalls durch eine Aminogruppe oder einen Stickstoff- oder Sauerstoffheterocyclus oder eine COOH- oder COOM-Gruppe substituiert sind; oder eine -CH₂-Hetero-cyclusgruppe, die 5 oder 6 Elemente aufweist und Stickstoff und/oder Sauerstoff enthält, steht;
R₂ für ein Wasserstoffatom, einen C₁- bis C₅-Alkylrest oder eine -CH₂-COO- (C₁-C₅) -Alkylgruppe steht
und R₃ für ein Wasserstoffatom, einen C₁- bis C₅-Alkyl- oder Alkenylrest, der gegebenenfalls durch -OH, eine Phosphatgruppe, einen Alkoxyrest, worin der Alkylrest 1 bis 3 Kohlenstoffatome umfaßt, oder Aryloxy substituiert ist; oder eine Alkylcarbonyloxygruppe (oder Arylcarbonyloxygruppe) steht;
oder auch R₂ und R₃ zusammen einen Ring mit 5 oder 6 Kohlenstoffatomen bilden; wobei R und R₃ unter Bildung eines Rings mit 5 bis 7 Atomen (Kohlenstoff, Sauerstoff, Schwefel) miteinander verbunden sein können;
- α für:
. entweder eine Einfachbindung steht, wenn A und A' für A1 und A'1 stehen oder wenn A und A' für A₂, d.h. eine -CHO- oder -COCH₃-Gruppe, stehen und B₂ und B'₂ für: 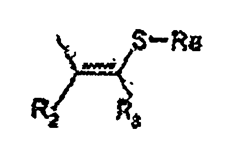 stehen,
. oder dann, wenn A und A' für A₂ stehen und B₂ und B'₂ für R₁ stehen, eine Gruppe der Formel: 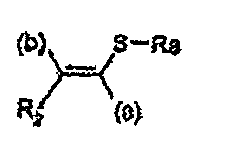 oder eine Gruppe der Formel: 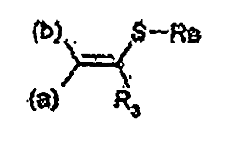 worin (a) für eine Bindung an Z steht und (b) für eine Bindung an das Stickstoffatom steht, steht,
- Z für einen C₆- bis C₂₁-Alkylrest, insbesondere C₁₂- oder C₁₃- bis C₂₁-Alkylrest steht, gegebenenfalls mit Einschub einer oder mehrerer Mehrfachbindungen und/oder eines oder mehrerer O- und/oder S-Heteroatome und/oder eines oder mehrerer aromatischer Ringe, und die pharmazeutisch unbedenklichen Salze dieser Verbindungen, mit der Maßgabe, daß R'₁ nicht für H oder einen C₁- oder C₂-Alkylrest steht, wenn n = 3 oder 4, R₁ für einen C₁- bis C₄-Alkylrest steht und Z für einen C₈- bis C₁₀-Alkylrest steht;
■■ wobei die Verbindung (II) der Formel: 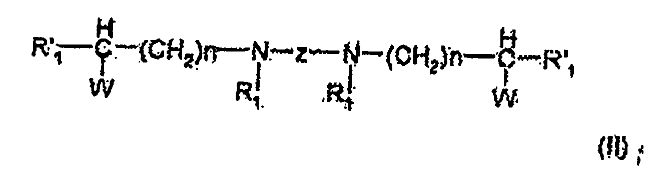 entspricht;
■■ wobei die Verbindung (III) der Formel: 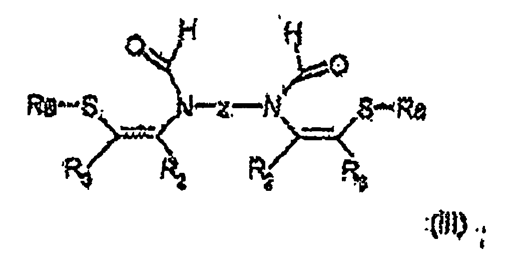 entspricht;
■■ wobei die Verbindung (IV) der Formel: 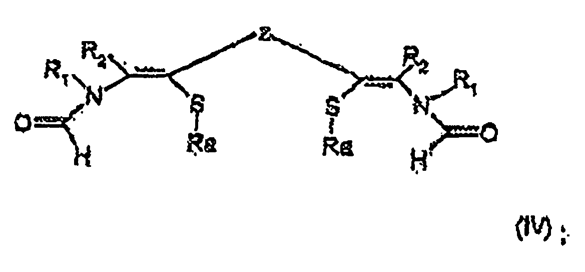 entspricht;
■■ wobei die Verbindung (V) der Formel: 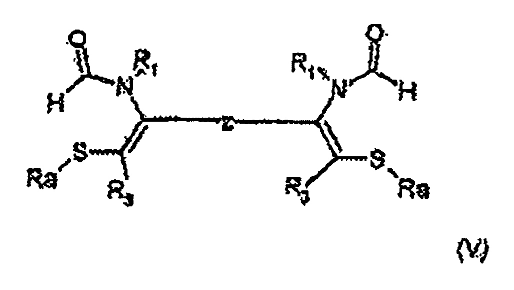 entspricht;
worin Ra, R₁, R'₁, R₂, R₃, W, n und Z die oben angegebene Bedeutung besitzen,
und
■ Artemisinin oder ein unter Artesunat, Artemether, Arteether, Hydroartemisinin oder Dihydroartemisinin ausgewähltes Derivat davon,
zur Verwendung bei der Behandlung und/oder Prävention von schwerer Malaria.

2. Kombination zur Verwendung nach Anspruch 1, **dadurch gekennzeichnet, daß** das Bisthiazoliumsalz der Formel (VI) ausgewählt ist unter:
- 1,12-Dodecamethylenbis[4-methyl-5-(2-hydroxyethyl)thiazolium]dibromid;
- 1,12-Dodecamethylenbis[4-methyl-5-(2-methoxyethyl)thiazolium]diiodid;
- 1,12-Dodecamethylenbis(4-methylthiazolium)-diiodid;
- 1,16-Hexadecamethylenbis[4-methyl-5-(2-methoxyethyl)thiazolium]diiodid;
- 3,10-Dioxadodecamethylenbis[5-(1,4-dimethyl)-thiazolium]diiodid;
- 3,10-Dioxadodecamethylenbis[5-(1-benzyl-4-methyl)thiazolium]dibromid;
- Dodecamethylenbis[5-(1-methyl-4-ethoxycarbonylethyl)thiazolium]diiodid;
- Dodecamethylenbis[5-(1-methyl-4-ethoxycarbonylethyl)thiazolium]dibromid;
- Dodecamethylenbis[4-(1-methyl)thiazolium]-diiodid;
- Dodecamethylenbis[4-(1-benzyl)thiazolium]-dibromid.

3. Kombination zur Verwendung nach den Ansprüchen 1 bis 2, **dadurch gekennzeichnet, daß** jeder der Wirkstoffe zur gleichzeitigen oder sequentiellen Verabreichung vorgesehen ist.

4. Pharmazeutische Zusammensetzung, umfassend therapeutisch wirksame Dosen von Artemisinin oder einem Derivat davon und mindestens eines Bisthiazoliumsalzes der Formel (VI) oder einer Vorstufe davon gemäß Anspruch 1 sowie mindestens einen pharmazeutisch unbedenklichen Hilfsstoff, zur Verwendung bei der Behandlung und/oder Prävention von schwerer Malaria.

5. Pharmazeutische Zusammensetzung zur Verwendung nach Anspruch 4, **dadurch gekennzeichnet, daß** es sich bei dem Bisthiazoliumsalz der Formel (VI) um 1,12-Dodecamethylenbis[4-methyl-5-(2-hydroxyethyl)thiazolium]dibromid handelt.

6. Pharmazeutische Zusammensetzung zur Verwendung nach Anspruch 4 oder Anspruch 5, **dadurch gekennzeichnet, daß** es sich bei dem Artemisininderivat um Artesunat handelt.

7. Pharmazeutische Zusammensetzung zur Verwendung nach einem der Ansprüche 4 bis 6, **dadurch gekennzeichnet, daß** sie zur oralen, rektalen oder injizierbaren Verabreichung und vorzugsweise zur intravenösen Verabreichung, insbesondere in Perfusionsform, geeignet ist.

8. Verwendung einer Kombination nach einem der Ansprüche 1 bis 3 zur Herstellung eines Arzneimittels zur Behandlung oder Prävention von schwerer Malaria.

9. Kit zur Verwendung bei der Behandlung oder Prävention von schwerer Malaria, umfassend einerseits mindestens ein Bisthiazoliumsalz der Formel (VI) oder eine Vorstufe davon und andererseits mindestens Artemisinin oder ein Derivat davon gemäß Anspruch 1, wobei die Verbindung der Formel (VI) oder eine Vorstufe davon und Artemisinin oder ein Derivat davon in verschiedenen Kompartimenten vorliegen und zur gleichzeitigen oder sequentiellen Verabreichung vorgesehen sind.

10. Kit zur Verwendung nach Anspruch 10, **dadurch gekennzeichnet, daß** es einerseits mindestens 1,12-Dodecamethylenbis[4-methyl-5-(2-hydroxyethyl)-thiazolium]dibromid und andererseits mindestens Artesunat umfaßt.
